# EUROPEAN PATENT APPLICATION

(11) **EP 1 433 721 A1**
(43) Date of publication of application: **30.06.2004**
(21) Application number: 02800262.4
(22) Date of filing: 27.09.2002
(51) Int. Cl.: B65D 85/16, A61F 13/15

(54) **DIAPER STORAGE FORMAT**

(30) Priority: 27.09.2001 JP 2001297802
(71) Applicant: UNI-CHARM CO., LTD., Kawanoe-shi Ehime 799-0111 (JP)
(72) Inventor: OTSUBO, Toshifumi, c/o UNI-CHARM Co. Ltd., Mitoyo-gun, Kagawa 769-1602 (JP)
(74) Representative: Sperling, Rüdiger, Dipl.-Ing.
(86) International application number: PCT/JP2002/010020
(87) International publication number: WO 2003/029106

(57) **Abstract**

A packaged assembly includes a package 1A formed in one of a rectangular hexahedron and a cube including first and second side walls opposed to each other and a plurality of disposable diapers 13, each including main body 25 having an absorbent core attached thereto and waist-surrounding peripheral end portions 19 of front and rear waist regions 17, 18 free from the absorbent core, packed within the package 1A so that the plurality of disposable diapers 13 may be placed one upon another between the first and second side walls. The package 1A contains therein first diapers 13a each having the waist-surrounding peripheral end portions 19 facing upward and second diapers 13b each having the waist-surrounding peripheral end portions facing downward and a plurality of diapers placed one upon another between the first and second side walls of the package constitute a group G in which the number of the first diapers 13a is equal to the number of the second diapers 13b.

## Description

### TECHNICAL FIELD OF THE INVENTION

This invention relates to a packaged assembly consisting of a plurality of disposable diapers orderly packed within a package so that these diapers may be placed one upon another between both side walls opposed to each other.

### BACKGROUND ART OF THE INVENTION

Japanese Patent Publication No. 2000-42028A discloses a packaged assembly consisting of a plurality of pants-type disposable diapers orderly packed within a package each comprising a liquid-pervious topsheet, a liquid-impervious backsheet and a liquid-absorbent core disposed between these two sheets. Within the package, these diapers are arranged so that front waist regions are opposed to rear waist regions for respective pairs of the adjacent diapers. In each of these diapers packed in this manner, a crotch region is folded toward the rear waist region of this diaper. Within the package, waist-surrounding peripheral end portions of the front and rear waist regions of the respective diapers occupy an upper packaging space of the package and the remaining portions of the respective diapers except for the waist-surrounding peripheral end portions occupy the lower packaging space defined below the upper packaging space.

In most of the disposable diaper, the liquid-absorbent core does not extend to the waist-surrounding peripheral end portions of the front and rear waist regions and therefore the remaining portion of the diaper except for the waist-surrounding peripheral end portions has a thickness larger than that of those waist-surrounding peripheral end portions. Particularly when the crotch regions of the respective diapers are folded toward the respective rear waist regions to pack the diapers in this manner of the prior art, the thickness of the remaining portions except for the waist-surrounding peripheral end portions of the waist regions will be remarkably larger than the thickness of these waist-surrounding peripheral end portions. This will lead to a remarkably large difference of the thickness between the portion of the diapers occupying the upper packaging space of the package and the remaining portions of the diapers occupying the lower packaging space of the package.

According to the packaged assembly as disclosed in the above-cited Publication, a total thickness of a group of diapers placed one upon another between both side walls opposed to each other of the package is larger in the lower packaging space than in the upper packaging space of the package. In other words, even if the diapers are packed so as to occupy the maximum dimension between both side walls opposed to each other of the package at its bottom, a gap will be left between each pair of the adjacent waist-surrounding peripheral end portions of the diapers and efficiency for packing the diapers within the package will be correspondingly deteriorated. According to this packing method of the prior art, the waist-surrounding peripheral end portions of the diapers occupying the upper packaging space of the package may be readily folded and make it difficult to stack vertically a plurality of the packages with a desired stability, i.e., the stack may tumble down. Furthermore, this packaged assembly of prior art is disadvantageous in that, when the groups of the diapers stacked vertically are stacked in the package, the groups of the diapers occupying the upper packaging space of the package tend to flatten or collapse the waist-surrounding peripheral end portions of the diapers occupying the lower packaging space of the package and may form these waist-surrounding peripheral end portions of the diapers with a plurality of irregular wrinkles.

An object of this invention is to provide a packaged assembly consisting of a plurality of disposable diapers efficiently packed within a package without anxiety that, when a plurality of such packaged assemblies are stacked vertically, such a stack of the packaged assemblies might be unintentionally tumbled down.

### DISCLOSURE OF THE INVENTION

According to this invention, there is provided a packaged assembly comprising a package formed of a flexible sheet in a hexahedron including first and second side walls opposed to each other and a plurality of disposable diapers, each including a main body having an absorbent core attached thereto and respective waist-surrounding peripheral end portions of front and rear waist regions free from the absorbent core, packed within the package so that the disposable diapers are placed one upon another between the first and second side walls of the package with the front and rear waist regions being opposed to each other in each of the diapers.

This invention further comprises the package containing therein first diapers each having the waist-surrounding peripheral end portions facing upward and second diapers each having the waist-surrounding peripheral end portions facing downward and the plurality of diapers placed one upon another between the first and second side walls of the package constitute a group in which the number of the first diapers is equal to the number of the second diapers.

According to one preferred embodiment of this invention, a difference between the number of the first diapers and the number of the second diapers is in a range of 0 - ± 3.

According to another preferred embodiment of this invention, the groups of at least two are stacked in a vertical direction within the package.

According to still another preferred embodiment of this invention, the groups of at least two are packed side by side within the package.

### BRIEF DESCRIPTION OF THE DRAWINGS

Fig. 1 is a partially cutaway perspective view showing a package;
Fig. 2 is a side view showing the package as diapers therein being exposed;
Fig. 3 is a partially cutaway perspective view showing the diaper;
Fig. 4 is a side view of a package illustrating one preferred embodiment of the packaged assembly comprising the diapers orderly packed within the package; and
Fig. 5 is a side view of a package illustrating another preferred embodiment of the packaged assembly comprising the diapers orderly packed within the package.

### DESCRIPTION OF THE BEST MODE FOR WORKING OF THE INVENTION

Details of the method according to this invention for orderly packing a plurality of diapers within a package will be more fully understood from the description given hereunder in reference to the accompanying drawings.

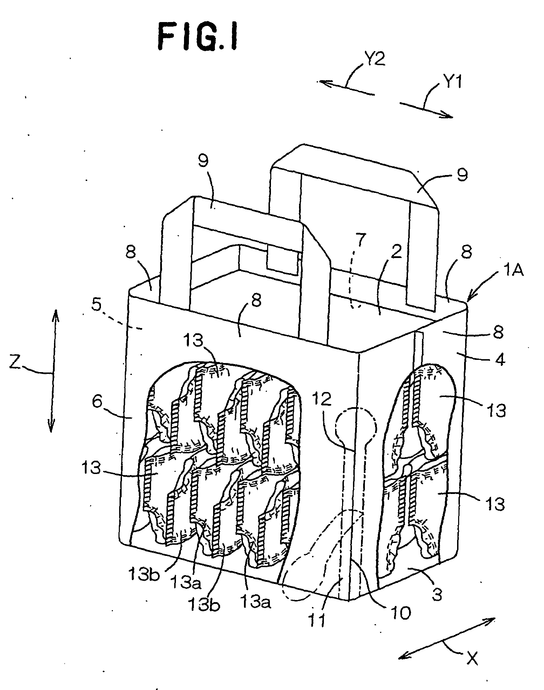

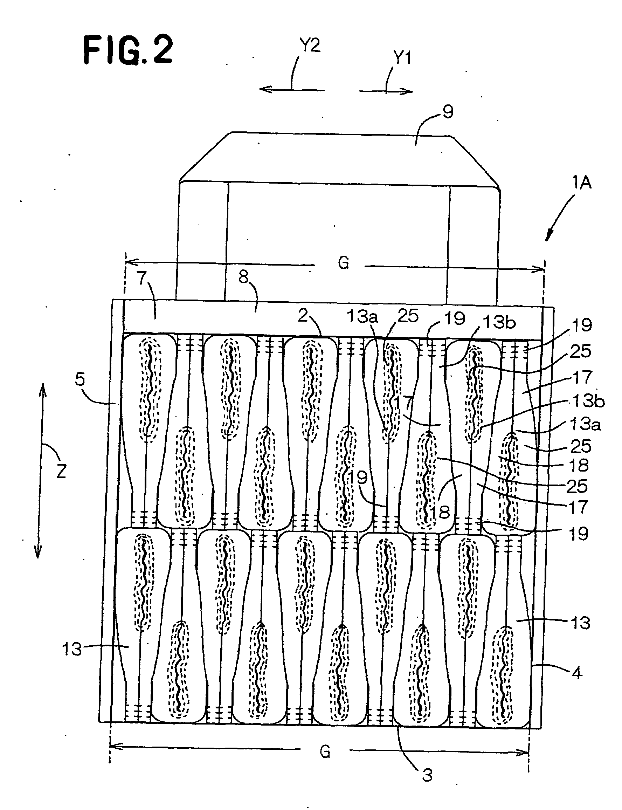

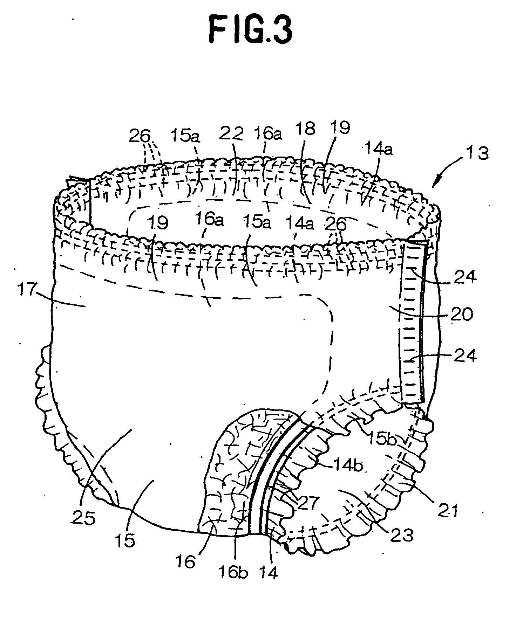

Fig. 1 is a partially cutaway perspective view showing a package 1A, Fig. 2 is a side view showing the package 1A with third side wall 6 thereof cutaway to expose diapers 13 packed therein and Fig. 3 is a partially cutaway perspective view showing the individual diaper 13 taken out from the package 1A. In Figs. 1 and 2, a transverse direction is indicated by an arrow X, a forward direction is indicated by an arrow Y1, a backward direction is indicated by an arrow Y2 and a vertical direction is indicated by an arrow Z. Term "inner surfaces" of a topsheet 14 and a backsheet 15 used herein should be understood to mean respective surfaces thereof facing a liquid-absorbent core 16 (absorbent member) and term "outer surfaces" of these sheets 14, 15 should be understood to mean respective surfaces thereof facing away from the core 16.

This packaged assembly comprises the package 1A and the pants-type disposable diapers 13 orderly packed within the package 1A, wherein the diapers 13 are arranged within the package 1A for the maximum packing efficiency. In this packaged assembly, a plurality of the diapers 13 are packed within the package 1A so as to be placed one upon another in one direction are packed under a compression.

Ten diapers 13 placed one upon another in the one direction constitute each group G and two such groups G are stacked in a vertical direction, as seen in Fig. 2. Two such stacks of the groups G are arranged side by side within the package 1A, i.e., totally four groups G of the diapers 13 are packed within this package 1A.

The package 1A is formed of a flexible sheet in a rectangular hexahedron being relatively long in the one direction in which each pair of adjacent surfaces are substantially orthogonal to each other. The package 1A may be in the form of a cube. The package 1A comprises substantially rectangular top and bottom walls 2, 3 opposed to each other, substantially rectangular first and second side walls 4, 5 vertically extending between respective opposite ends of the top and bottom walls 2, 3, and substantially rectangular third and fourth side walls 6, 7 vertically extending between respective opposite side edges of the top and bottom walls 2, 3. These side walls 4, 5, 6, 7 respectively have extended walls 8 above the opposite ends and the opposite side edges of the top wall 2. The extended walls 8 of the third and fourth side walls 6, 7 are provided with a pair of handling straps describing circular arcs extending upward from the package 1A.

A corner 10 at which the second side wall 5 crosses the third side wall 6 is formed with a perforated line 11 extending vertically. As indicated by an imaginary line in Fig. 1, a region 12 enclosed by the perforated line 11 so as to extend in the second and third side walls 5, 6 may be torn off to form a dispensing outlet for the individual diaper 13 along the corner 10.

As will be seen in Fig. 3, the diaper 13 comprises a liquid-pervious topsheet 14 facing a wearer's body, a liquid-impervious backsheet 15 facing away from wearer's body and a liquid-absorbent core 16 interposed between these two sheets 14, 15. The core 16 is bonded to the inner surface of at least one of the top- and backsheets 14, 15.

The core 16 comprises a mixture of fluff pulp and super-absorbent polymer particles or a mixture of fluff pulp, super-absorbent polymer particles and thermoplastic synthetic resin fibers, in any case, compressed to a desired thickness. Necessarily, thickness of the core 16 is larger than those of the top- and backsheets. Preferably, the core 16 is entirely covered with a liquid-pervious sheet such as tissue paper or hydrophilic fibrous nonwoven fabric in order to prevent it from getting out of shape and/or to prevent the polymer particles from falling off.

The diaper 13 has front and rear waist regions 17, 18 opposed to each other. In the diaper 13, transversely opposite side portions 20 of the respective waist regions extending in the longitudinal direction are overlaid and joined together in the vicinity of outermost edges of the respective side edge portions 20 by means of heat-sealing lines 24 arranged intermittently in the longitudinal direction. The diaper 13 is formed with a waist-hole 22 and a pair of leg-holes 23 lying below the waist-hole 22.

In the diaper 13, longitudinally opposite ends 16a of the core 16 lie inside waist-surrounding peripheral end portions of the front and rear waist regions 17, 18 and transversely opposite side edges 16b of the core 16 lie inside the transversely opposite side portions 20 of the waist regions and thigh-surrounding peripheral end portions 21. In the diaper 13, the waist-surrounding peripheral end portions 19 of the front and rear waist regions, the transversely opposite side portions 20 of the waist regions and the thigh-surrounding peripheral end portions 21 are free from the presence of the core 16, so a main body 25 which is a region of the diaper 13 except for these portions 19, 20, 21 has thickness larger than those of these portions 19, 20, 21. The individual diaper 13 is folded in two so that the front and rear waist regions 17, 18 may by placed upon each other as this diaper 13 is packed within the package 1A.

In the group G, first diapers 13a each having the waist-surrounding peripheral end portions 19 of the respective waist regions lying above the main body 25 and second diapers 13b each having the waist-surrounding peripheral end portions 19 of the respective waist regions lying below the main body 25 are alternately placed one upon another between first and second side walls 4, 5. In the group G, the number of the first diapers 13a is five and the number of the second diaper 13b is also five, that is, the numbers of the first and second diapers 13a, 13b are the same. From the first side wall 4 toward the second side wall 5 in one direction, the front waist region 18 of a second diaper 13b lying immediately behind the foremost first diaper 13a is placed upon the rear waist region 18 of this foremost first diaper 13a and then the front waist region 17 of the next first diaper 13a lying immediately behind the next second diaper 13b is placed upon the rear waist region 18 of this second diaper 13b, and so on.

In this packaged assembly, the first diaper 13a and the second diaper 13b are alternately placed one upon another between the first and second side walls 4, 5 and the number of the first diaper 13a is equal to the number of the second diaper 13b in the group G. Therefore, the thickness of the group G between the first and second side walls 4, 5 has substantially no difference between its upper half and lower half in spite of the fact that, in both the first diapers 13a and the second diapers 13b, the thickness of the main body 25 is larger than the thickness of the waist-surrounding peripheral end portions 19. Even when the diapers 13 are packed within the package 1A to fill the maximum dimension between the first and second side walls 4, 5 of the package 1A, no gap is formed between each pair of the diapers 13 and thereby a packing efficiency for the diapers 13 within the package 1A can be improved.

In this packaged assembly, no gap is formed between each pair of the adjacent diapers 13 both in the upper part and in the lower part of the group G, so a plurality of packages 1A can be stably stacked in a vertical direction without anxiety that such stack of the packaged assemblies might be unintentionally tumbled down because the lower package 1A can reliably support the upper package 1A.

In this packaged assembly, the first diapers 13a and the second diapers 13b in the group G are placed one upon another between the first and second side walls 4, 5 of the package 1A in sufficiently close contact one with another to prevent the waist-surrounding peripheral end portions 19 of the diapers 13 from being easily folded. Therefore, even when two groups G are stacked in a vertical direction within the package 1A, it is not apprehended that the upper group G of the diapers 13 might flatten or collapse the waist-surrounding peripheral portions 19 of the diapers 13 in the lower group G and consequently form the waist-surrounding peripheral end portions of the diapers 13 in the lower group G with a plurality of irregular wrinkles.

Along the waist-surrounding peripheral end portions 19 of the diaper 13, respective end portions 14a, 15a of the top- and backsheets 14, 15 extending outward beyond longitudinally opposite ends 16a of the core 16 are overlaid and joined together. The waist-surrounding peripheral end portions 19 of the diaper 13 are provided with waist-surrounding elastic members 26 secured thereto in a stretched state. These waist-surrounding elastic members 26 are interposed between the top- and backsheets 14, 15 and bonded to respective inner surfaces of the end portions 14a, 15a of these sheets 14, 15.

Along the transversely opposite side portions 20 of the waist regions and thigh-surrounding peripheral end portions 21, respective side edge portions 14b, 15b of the top- and backsheets 14, 15 extending outward beyond transversely opposite side edges 16b of the core 16 are overlaid and joined together. The thigh-surrounding peripheral end portions 21 of the diaper 13 are provided with thigh-surrounding elastic members 27 secured thereto in a stretched state. These thigh-surrounding elastic members 27 are interposed between the top- and backsheets 14, 15 and bonded to respective inner surfaces of the side edge portions 14b, 15b of these sheets 14, 15.

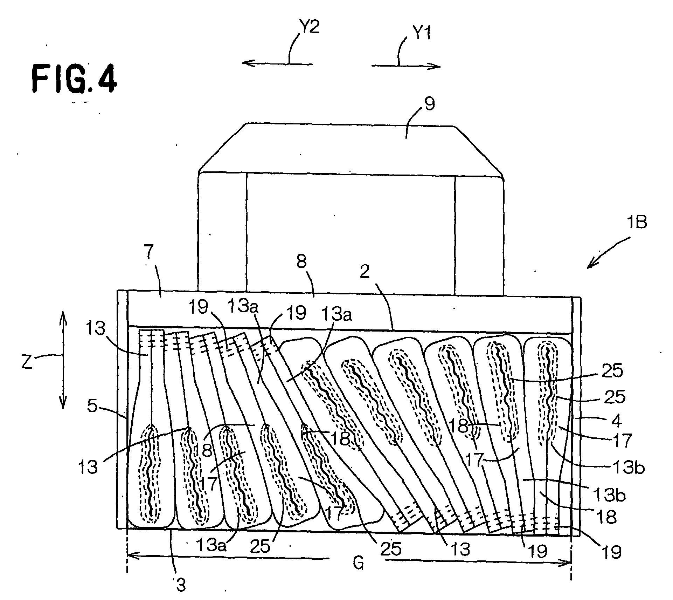

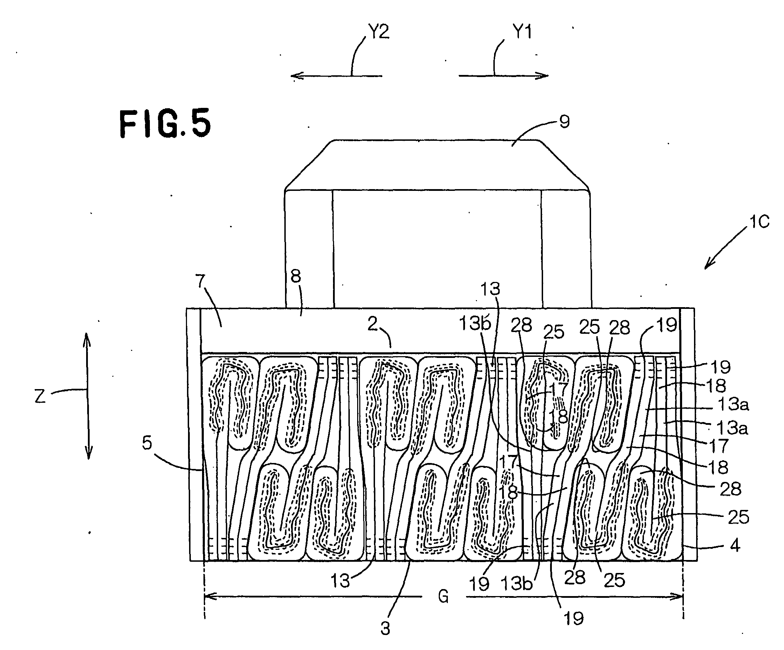

Fig. 4 is a side view of a package 1B illustrating one preferred embodiment of the packaged assembly and Fig. 5 is a side view of the package 1C illustrating another preferred embodiment of the packaged assembly. Figs. 4 and 5 show the packages 1B, 1C with the third side walls 6 thereof cutaway to expose the diapers 13 packed therein. In Figs. 4 and 5, a forward direction is indicated by an arrow Y1, a backward direction is indicated by an arrow Y2 and a vertical direction is indicated by an arrow Z.

Similarly to the package 1A shown in Fig. 1, each of packages 1B, 1C is formed of a flexible sheet in a rectangular hexahedron being relatively long in the one direction in which each pair of adjacent surfaces are substantially orthogonal to each other. The diaper 13 is the pants-type disposable diaper 13 similar to that shown in Fig. 1 and the main body 25 which is the region of the diaper 13 except for the waist-surrounding peripheral end portions 19, the transversely opposite side portions 20 of the waist regions and the thigh-surrounding peripheral end portions 21 has the thickness larger than those of these portions 19, 20, 21. The individual diapers 13 are folded so that the front and rear waist regions 17, 18 thereof may contact each other as these diapers 13 are packed within the package 1B, 1C.

The packaged assemblies shown in Figs. 4 and 5, respectively, are distinguished from the packaged assembly shown in Fig. 1 in arrangement as will be described. In the packaged assembly shown in Fig. 4, one group G consists of eleven diapers 13 placed one upon another between the first and second side wall 4, 5 of the package 1B. In the packaged assembly shown in Fig. 5, one group G consists of twelve diapers 13 placed one upon another between the first and second side walls 4, 5 of the package 1C. Within these packages 1B, 1C, these diapers 13 are under compression in the direction from the first side wall 4 toward the second side wall 5.

Referring to Fig. 4, the group G comprises five first diapers 13a and six second diapers 13b. In the group G, the second diapers 13b are placed one upon another from the first side wall 4 of the package 1B toward the middle and the first diapers 13a are placed one upon another from the middle in back and forth direction toward the second side wall 5.

The waist-surrounding peripheral end portions 19 of the first diaper 13a lie above the main body 25 which is the region except for the waist-surrounding peripheral end portions 19. The waist-surrounding peripheral end portions 19 of the second diaper 13b lie below the main body 25. Concerning the first diapers 13a arranged from the middle of the package 1B toward the second side wall 5 of the package 1B, the front waist region 17 of the diaper 13a lying immediately behind the forehand first diaper 13a is placed upon the rear waist region 18 of this forehand first diaper 13a and the front waist region 17 of the first diaper 13a lying immediately behind this first diaper 13a is placed upon the rear waist region 18 of this precedent first diaper 13a and so on. Concerning the second diapers 13b arranged from the first side wall 3 of the package 1B toward the middle of the package 1B, the front waist region 17 of the diaper 13b lying immediately behind the forehand second diaper 13b is placed upon the rear waist region 18 of this forehand second diaper 13b and the front waist region 17 of the second diaper 13b lying immediately behind this second diaper 13b is placed upon the rear waist region 18 of this precedent second diaper 13b, and so on.

In the packaged assembly shown in Fig. 4, the number of the second diapers 13b is larger than the number of the first diapers 13a by one in the group G. In spite of this difference in the number of the first and second diapers 13a, 13b, there is no anxiety that a gap might be formed between each pair of the adjacent diapers 13 both in the upper part and in the lower part of the group G because the thickness of the group G in back and forth direction in the vicinity of the top wall 2 of the package 1B is substantially equal to that in the vicinity of the bottom wall 3 of the package 1B. In this way, a packing efficiency for the diapers 13 within the package 1B is improved. Gap-free packing achieved by this embodiment allows a plurality of the packages 1B to be stably stacked in a vertical direction without apprehension that the stack might be unintentionally tumbled down.

Referring to Fig. 5, one group G consists of six first diapers 13a and six second diapers 13b. In the group G, each pair of the first diapers 13a are placed upon each other and, behind this pair of the first diapers 13a, each pair of the second diapers 13b placed upon each other between the first and second side walls 4, 5. In this manner, the pair of the first diapers 13a and the pair of the second diapers 13b are alternately placed one upon another between the first and second side walls 4, 5.

Concerning the first diaper 13a, a crotch region 28 is folded toward the outer surface of the backsheet 15 extending in the rear waist region 18 and the waist-surrounding peripheral end portions 19 lie above the main body 25 except for these waist-surrounding peripheral end portions 19. Concerning the second diaper 13b, the crotch region 28 is folded toward the outer surface of the backsheet 15 extending in the rear waist region 18 and the waist-surrounding peripheral end portions 19 lie below the main body 25. In each pair of the first diapers 13a, the front waist region 17 of the rear diaper 13a is placed upon the rear waist region 18 of the front diaper 13a. In each pair of the second diapers 13b, the rear waist region 18 of the rear diaper 13b is placed upon the front waist region 17 of the front diaper 13b. The rear waist region 18 of the first diaper 13a and the rear waist region 18 of the second diaper 13b are placed upon each other.

In the packaged assembly shown in Fig. 5, the number of the first diapers 13a is equal to the number of the second diapers 13b and there is no difference in the thickness of the package 1C between the first and second side walls 4, 5 between in the vicinity of the top wall 2 and in the vicinity of the bottom wall 3. In this packaged assembly, no gap is formed between each pair of the adjacent diapers both in the upper part and in the lower part of the package 1C and a packing efficiency for the diapers 13 within the package 1C is effectively improved. Such a gap-free packing allows a plurality of packages 1C to be stably stacked in a vertical direction without anxiety that such a stack of the packages IC might be unintentionally tumbled down.

The packaged assembly shown in Fig. 5 may be modified so that a set of three or more first diapers 13a and a set of three or more second diapers 13b are alternately placed one upon another between the first and second side walls 4, 5. In the packaged assembly shown in Fig. 5, it is not essential to fold the crotch regions 28 of the diapers 13.

For these packaged assemblies as have been described above, a difference in the number of the first diapers 13a and the number of the second diapers 13b in one and same group G is preferably in a range of 0 - ±3. In any one of these embodiments, three or more groups G may be stacked in a vertical direction and/or three or more groups G may be placed side by side within the package 1A, 1B, 1C. The diapers packed within the package 1A, 1B, 1C are not limited to the pants-type diaper but may be open-type diapers. The number of the diapers 13 packed within the package 1A, 1B, 1C is not specified and may be increased to thirteen or more or reduced to nine or less.

While the diapers are placed one upon another in the direction from the first side wall 4 toward the second side wall 5, it is possible that the diapers are placed upon another in the direction from the third side wall 6 toward the fourth side wall 7. Correspondingly, the transverse direction may be defined by a direction from the first side wall 4 toward the second side wall 5.

The package 1A, 1B, 1C may be formed using a breathable but liquid-impervious plastic film or a breathable but liquid-impervious fibrous nonwoven fabric. The topsheet 14 of the diaper 13 may be formed using a hydrophilic fibrous nonwoven fabric, a hydrophobic fibrous nonwoven fabric having a plurality of fine pores or a plastic film having a plurality of fine pores. The backsheet 15 of the diaper 13 may be formed using a hydrophobic fibrous nonwoven fabric, a breathable but liquid-impervious plastic film, a composite nonwoven fabric consisting of laminated one with one upon another or a composite sheet consisting of a hydrophobic fibrous nonwoven fabric and a breathable but liquid-impervious plastic film.

The plastic film used herein is preferably made of polyolefine-based thermoplastic synthetic resin such as polypropylene or polyethylene.

The fibrous nonwoven fabric used herein may be selected from a group including produces obtained by spun lace process, needle punch process, melt blown process, thermal bond process, spun bond process, chemical bond process and air-through process. Component fiber of the nonwoven fabric may be selected from a group including polyolefine-, polyester-, polyamide-based fibers and core-sheath type or side-by-side type conjugated fibers of polyethylene/polypropylene or polyethylene/polyester.

The packaged assembly according to this invention has the advantageous effects that a plurality of diapers comprising the first diapers each having the waist-surrounding peripheral end portions lying above the main body except for the waist-surrounding peripheral end portions and the second diapers each having the waist-surrounding peripheral end portions lying below the main body are packed within the package and each group of the diapers consists of the first and second diapers of approximately same number. Even if a plurality of the diapers each having different thickness between the waist-surrounding peripheral end portions and the main body are packed within the package so as to fill the maximum dimension between the first and second side walls of the package, no gap will be formed between each pair of the adjacent diapers both in the upper part and in the lower part of the group. In this way, a packing efficiency for the diapers within the package is improved.

A gap-free packing achieved by this packaged assembly allows a plurality of the packages to be stably stacked in a vertical direction without apprehension that such a stack might be unintentionally tumbled down.

With this packaged assembly, the first and second diapers are placed one upon another in sufficiently close contact one with another between the first and second side walls to prevent the waist-surrounding peripheral end portions to be easily folded. Therefore, even when two or more groups are stacked in a vertical direction within the package, it is not apprehended that the upper group(s) of the diapers might flatten the waist-surrounding peripheral portions of the diapers in the lower group(s) and consequently form the waist-surrounding peripheral end portions of the diapers in the lower group(s) with a plurality of irregular wrinkles.

Even when the number of the first diapers is not equal to the number of the second diapers in one and same group, no gap will be formed between each pair of the adjacent diapers both in the upper part and in the lower part of the group so far as the difference between these numbers is in a range of 0 - ±3. In this way, a packing efficiency for the diapers within the package is improved.

## Claims

1. A packaged assembly comprising a package formed of a flexible sheet in a hexahedron including first and second side walls opposed to each other and a plurality of disposable diapers, each including a main body having an absorbent core attached thereto and leave respective waist-surrounding peripheral end portions of front and rear waist regions free from the absorbent core, packed within said package so that said disposable diapers are placed one upon another between said first and second side walls of said package with said front and rear waist regions being opposed to each other in each of the diapers, said packaged assembly further comprising:
said package containing therein first diapers each having said waist-surrounding peripheral end portions facing upward and second diapers each having said waist-surrounding peripheral end portions facing downward and said plurality of diapers placed one upon another between said first and second side walls of said package constitute a group in which the number of said first diapers is equal to the number of said second diapers.

2. The packaged assembly according to Claim 1, wherein a difference between the number of said first diapers and the number of said second diapers is in a range of 0 - ±3.

3. The packaged assembly according to Claim 1 or 2, wherein said groups of at least two are stacked in a vertical direction within said package.

4. The packaged assembly according to any one of Claims 1 - 3, wherein said groups of at least two are packed side by side within said package.
